# EUROPEAN PATENT APPLICATION

(11) **EP 0 636 612 A1**
(43) Date of publication of application: **01.02.1995**
(21) Application number: 93914965.4
(22) Date of filing: 07.07.1993
(51) Int. Cl.: C07D 217/26

(54) **PROCESS FOR PRODUCING TETRAHYDROISOQUINOLINE-3-CARBOXYLIC ACID DERIVATIVE**

(30) Priority: 31.08.1992 JP 231043/92
(71) Applicant: KYOWA HAKKO KOGYO CO., LTD., Chiyoda-ku, Tokyo 100 (JP)
(72) Inventor: KUGE, Yukihiro, Kawachinagano-shi, Osaka 586 (JP); SUGAYA, Toru, Nara-shi, Nara 631 (JP); TOMIOKA, Shinji, Hashimoto-shi, Wakayama 649-73 (JP)
(74) Representative: Casalonga, Axel
(86) International application number: JP9300933
(87) International publication number: WO9405640

(57) **Abstract**

A process for producing a 1,2,3,4-tetrahydroisoquinoline-3-carboxylic acid derivative, represented by general formula (II), or a salt thereof (wherein R¹, R² and R³ may be the same or different from each other and each represents hydrogen, hydroxy or lower alkoxy; and R⁴ represents hydrogen, lower alkyl, aryl or aralkyl) by the reaction of a phenylalanine derivative represented by general formula (I) with formaldehyde or paraformaldehyde in the presence of sulfuric or hydrobromic acid, wherein R¹, R², R³ and R⁴ are each as defined above.

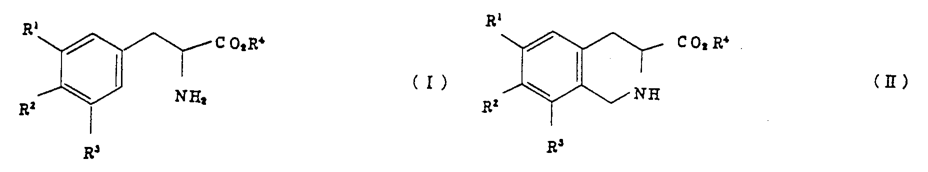

## Description

### Technical Field

The present invention relates to a process for safely producing 1,2,3,4-tetrahydroisoquinoline-3-carboxylic acid derivatives useful as medical intermediates.

### Prior Art

A process for producing 1,2,3,4-tetrahydroisoquinoline-3-carboxylic acid derivatives by allowing phenylalanine derivatives to react with formaldehyde or paraformaldehyde in the presence of an acid is known as the Pictet Spengler reaction [Journal of Organic Chemistry, 16, 430 (1951), Indian Journal of Chemistry, 13, 230 (1975), Chemical and Pharmaceutical Bulletin, 31(1), 312 (1983), and Japanese Published Unexamined Patent Application Nos. 16865/82 and 193969/90].

In these known methods, hydrochloric acid is used as an acid catalyst. However, in this case, it has been known that bis(chloromethyl)ether, which is a strong mutagen [IARC Monographs, 4, 231 (1974)], is formed through the reaction of hydrochloric acid with formaldehyde or paraformaldehyde [Organic Synthesis Coll. Vol. 4, 101-103 (1963)].

Further when hydrochloric acid is used as an acid catalyst, the resultant product of the Pictet Spengler reaction using L-phenylalanine as a substrate has an optical purity of 50 to 85 % ee and accompanies a high degree of racemization occurs. (Japanese Published Unexamined Patent Application No. 193969/90).

### Disclosure of the Invention

The present invention relates to a process for safely producing 1,2,3,4-tetrahydroisoquinoline-3-carboxylic acid derivatives [hereinafter referred to as "Compound (II)"] or salts thereof represented by the following formula (II): 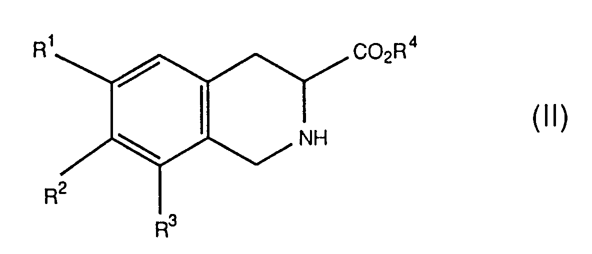
wherein R¹, R², and R³ independently represent hydrogen, hydroxyl or lower alkoxyl, and R⁴ represent hydrogen, lower alkyl, aryl or aralkyl, the process comprising, allowing phenylalanine derivatives to react with formaldehyde or paraformaldehyde in the presence of sulfuric acid or hydrobromic acid, the phenylalanine derivatives being represented by the following formula (I): 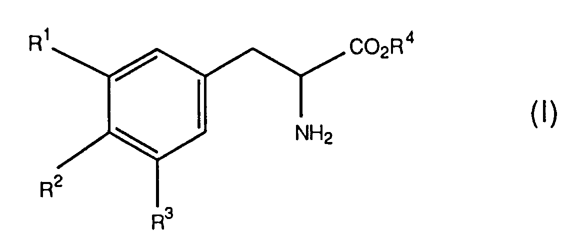
wherein R¹, R², R³ and R⁴ have the same meaning as defined above.

In the definition of R¹, R² and R³, the lower alkyl and the alkyl moiety in the lower alkoxy mean a straight chain or branched alkyl group having 1 to 6 carbon atoms such as methyl, ethyl, propyl, isopropyl, butyl, isobutyl, sec-butyl, tert-butyl, pentyl, isopentyl, neopentyl and hexyl. The aryl means phenyl or naphthyl. The aralkyl means benzyl, phenetyl or trityl each having 7 to 20 carbon atoms. The salts of Compound (II) include sulfates and hydrobromides.

The process for producing Compound (II) is described below.

Compound (II) can be obtained by allowing Compound (I) to react with formaldehyde or paraformaldehyde in an aqueous sulfuric acid solution or hydrobromic acid.

The concentration of the aqueous sulfuric acid solution is in the range of 0.001 to 12 N, preferably 0.01 to 1 N.

The concentration of solution of hydrobromic acid is in the range of 20 to 50%.

Compound (I) is synthesized by the method disclosed in, for example, Journal of American Chemical Society, 73, 5386 (1951).

As formaldehyde, 37 to 40 % formalin is generally used in an amount of 1 to 10 equivalents, preferably 1.5 to 2 equivalents, based on Compound (I). This applies to the case of paraformaldehyde.

The reaction is carried out at 40 to 100°C. The reaction time is long when the temperature is low, and the reaction time is short when the temperature is high. With the use of the optically active Compound (I), high temperature promotes racemization. Therefore, the reaction is preferably carried out at 50 to 80°C and completed in 0.5 to 36 hours, generally in 3 to 12 hours.

Compound (II) is generally obtained in the form of its salt. When it is desired to obtain Compound (I) in its free form, the Compound (II) may be dissolved or suspended in an alkali aqueous solution and an acid is added thereto for neutralization. Compound (I) thus formed in its free form may be isolated and purified.

Compound (II) obtained by the above production method can be isolated and purified by purification methods conventionally used in organic synthetic chemistry, for example, filtration, extraction, washing, drying, concentration, recrystallization, and various kinds of chromatography.

Compound (II) obtained in the present invention can be used as a synthetic intermediate for N-methyl-D-aspartic acid receptor antagonists disclosed in, for example, Journal of Organic Chemistry, 56, 4388 (1991).

Certain embodiments of the present invention are described by referring to the following examples.

### Best Mode for Carrying out the Invention

In the following examples, optical purity was measured by high performance liquid chromatography (HPLC) under the two types of conditions:

### a) Example 2

- Column;: SUMICHIRAL OA-5000(L) (Sumitomo Chemistry), 4.6 ⌀ x 150 mm
- Mobile phase;: 2 mM CuSO₄ solution:acetonitrile = 85:15
- Detection UV;: 254 nm
- Column temperature;: 20°C
- Flow rate;: 1.0 ml/min

### b) Example 3

- Column;: CROWNPAK (+) (Daicel Chemistry), 4.6 ⌀ x 150 mm
- Mobile phase;: HClO₄ aqueous solution (pH 2.0)
- Detection UV;: 254 nm
- Column temperature;: 20°C
- Flow rate;: 0.8 ml/min

### Example 1

### (±)-6-Hydroxy-1,2,3,4-tetrahydroisoquinoline-3-carboxylic acid (Compound 1)

To 61.5 g of D, L-m-tyrosine suspended in 250 ml of 0.05 N sulfuric acid aqueous solution, 48 ml of 37 % formalin aqueous solution was added dropwise. The reaction mixture was stirred at 70°C for 12 hours, and subjected to crystallization under ice cooling for 2 hours. The obtained crystal was filtered off, and dried at 60°C under reduced pressure to obtain 65.6 g of Compound 1 (yield 100 %). Compound 1 thus obtained was dissolved in an aqueous solution of 2 N sodium hydroxide, and was then adjusted to pH 5 with concentrated hydrochloric acid. The precipitate was filtered off, washed with water and dried at 60°C under reduced pressure to obtain 52.5 g of purified product of Compound 1.

¹H-NMR(D₂O-NaOD) δ (ppm): 6.62(1H,d,J=8.1Hz), 6.23(1H,d,J=8.1Hz), 6.19(1H,s), 3.65(1H,d,J=15.2Hz), 3.57(1H,d,J=15.2Hz), 3.13(1H,dd,J=11.0, 3.6Hz), 2.68(1H,dd,J=16.2, 3.6Hz), 2.51(1H,d,J=16.2, 11.0Hz)

### Example 2

### (-)-1,2,3,4-Tetrahydroisoquinoline-3-carboxylic acid hydrobromide (Compound 2)

To 10 g of L-phenylalanine suspended in 108 ml of 47 % hydrobromic acid, 23 ml of 37 % formalin aqueous solution was added dropwise. The reaction mixture was stirred at 65°C for 7 hours, and subjected to crystallization under ice cooling for 3 hours. The precipitate was filtered off, and dried at 55°C under reduced pressure to obtain 13.5 g of Compound 2 (yield 86.4 %, optical purity 97 %ee).

¹H-NMR(d₆-DMSO) δ (ppm): 9.76(1H,br), 7.41(4H,m), 4.60(1H,dd,J=11.3, 5.1Hz), 4.49(2H,s), 3.46(1H,dd,J=17.0, 5.1Hz), 3.26(1H,dd,J=17.0, 11.2Hz)

### Example 3

### (-)-6-Hydroxy-1,2,3,4-tetrahydroisoquinoline-3-carboxylic acid (Compound 3)

In the same manner as in Example 1, 61.5 g of L-m-tyrosine having an optical purity of 100 %ee was allowed to react to obtain 62.3 g of Compound 3 (yield 95 %, optical purity 100 % ee).

### Industrial Applicability

The present invention provides a process for safely producing 1,2,3,4-tetrahydroisoquinoline-3-carboxylic acid derivatives, which are useful as medical intermediates, in a large amount without producing a mutagenic substance.

## Claims

1. A process for producing 1,2,3,4-tetrahydroisoquinoline-3-carboxylic acid derivatives or salts thereof represented by the following formula (II): 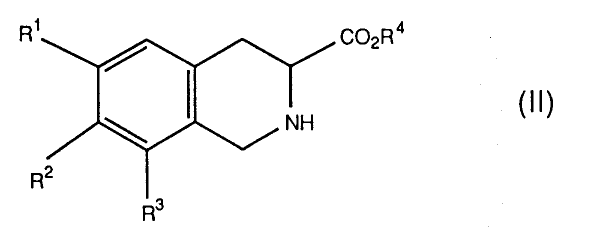 wherein R¹, R² and R³ independently represent hydrogen, hydroxyl or lower alkoxyl, and R⁴ represent hydrogen, lower alkyl, aryl or aralkyl, the process comprising, allowing phenylalanine derivatives to react with formaldehyde or paraformaldehyde in the presence of sulfuric acid or hydrobromic acid, the phenylalanine derivatives being represented by the following formula (I): 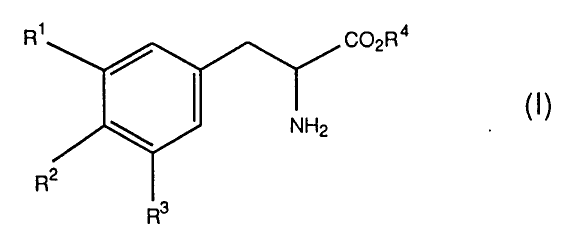 wherein R¹, R², R³ and R⁴ have the same meaning as defined above.
